**Europäisches Patentamt**

**European Patent Office**

**Office européen des brevets**

(11) Veröffentlichungsnummer: **0 511 516 B1**

(12) # EUROPÄISCHE PATENTSCHRIFT

(45) Veröffentlichungstag der Patentschrift: **04.10.95**

(51) Int. Cl.[6]: **C07K 14/39**, C12P 21/08, C12Q 1/04

(21) Anmeldenummer: **92105755.0**

(22) Anmeldetag: **03.04.92**

(54) **Schizosaccharomyces-spezifische Polypeptide.**

(30) Priorität: **29.04.91 DE 4113949**

(43) Veröffentlichungstag der Anmeldung:
**04.11.92 Patentblatt 92/45**

(45) Bekanntmachung des Hinweises auf die
Patenterteilung:
**04.10.95 Patentblatt 95/40**

(84) Benannte Vertragsstaaten:
**AT BE CH DE DK ES FR GB GR IT LI LU NL PT
SE**

(56) Entgegenhaltungen:
**EP-A- 0 385 391**

**NUCLEIC ACIDS RESEARCH. Bd. 16, Nr. 17,
12. September 1988, ARLINGTON, VIRGINIA
US Seiten 8603 - 8617 B.KUDLA ET AL. 'Con-
struction of an expression vector for the
fission yeast Schizosaccharomyces pombe'**

**CURRENT MICROBIOLOGY Bd. 22, Nr. 6, Juni
1991, NEW YORK Seiten 339 - 343 M.BRöKER
'Identification of the yeast genus Schizosaccharomyces by a murine monoclonal antibody'**

(73) Patentinhaber: **BEHRINGWERKE Aktiengesellschaft
Postfach 1140
D-35001 Marburg (DE)**

(72) Erfinder: **Bröker, Michael
Pappelweg 30
W-3550 Marburg (DE)**
Erfinder: **Hock, Johann
Haselhecke 8
W-3550 Marburg (DE)**

**Beschreibung**

Die Erfindung betrifft neue Schizosaccharomyces spezifische Proteine und Antikörper dagegen. Diese Proteine und Antikörper können zur Identifizierung der Hefegattung Schizosaccharomyces verwendet werden.

Die Spalthefe Schizosaccharomyces enthält nach Slooff (W. C. Slooff (1970) "Genus 19. Schizosaccharomyces" in The Yeast. A taxonomic study. The North Holland Publishing Co. Amsterdam, pp. 733-755) vier Arten: S. pombe, S. malidevorans, S. octosporus und S. japonicus. S. japonicus ist wiederum in S. japonicus var. japonicus und S. japonicus var. versatilis unterteilt. Diese Taxonomie ist jedoch nicht allgemein anerkannt und es wird diskutiert, ob nicht zusätzliche Gattungsnamen, wie Octosporomyces oder Hasegawaea eingeführt werden sollen.

Zur Identifizierung und Klassifizierung von Hefen werden im allgemeinen neben morphologischen Kriterien insbesondere physiologische Eigenschaften, wie die Verwertung von Mono-, Di- und Trisacchariden, die Verwertung von Nitrat und der Bedarf an bestimmten Vitaminen herangezogen. Die Identifizierung auf Grund dieser Merkmale ist jedoch nicht immer eindeutig und kann auch zu Fehlbestimmungen führen.

Es wurde nun gefunden, daß bestimmte Proteine, die von dem murinen monoklonalen Antikörper mAK JHF13-17 erkannt werden, für Schizosaccharomyces spezifisch sind.

Das Hybridom, das den vorgenannten mAK JHF13-17 sezerniert, wurde am 6.3.91 bei der Deutschen Sammlung von Mikroorganismen und Zellkulturen G.M.G.H. (DMS) gemäß Budapester Vertrag unter der Nummer DSM ACC 2005 hinterlegt.

Die Erfindung betrifft daher:

1. Polypeptide oder immunogene Teile davon aus Schizosaccharomyces, welche von dem monoklonalen Antikörper JHF13-17 erkannt werden.

2. Monoklonalen Antikörper JHF13-17.

3. Monoklonale oder polyklonale Antikörper gegen Polypeptide oder immunogene Teile aus Schizosaccharomyces, dadurch gekennzeichnet, daß die Polypeptide oder immunogene Teile davon von dem monoklonalen Antikörper JHF13-17 erkannt werden.

4. Verfahren zur Herstellung von polyklonalen Antikörpern, dadurch gekennzeichnet, daß

a) Polypeptide oder immungene Teile davon, welche von dem monoklonalen Antikörper JHF13-17 erkannt werden, isoliert werden und

b) ein geeigneter Organismus mit den Polypeptiden oder immunogenen Teilen aus Schritt a) immunisiert wird.

5. Verfahren zur Identifizierung von Schizosaccharomyces, dadurch gekennzeichnet, daß Schizosaccharomyces zusammen mit Antikörpern gegen Polypeptide oder immunogene Teile davon nach Anspruch 1 inkubiert wird.

6. Verwendung von Antikörpern gegen Polypeptide oder immunogene Teile davon, welche von dem monoklonalen Antikörper JHF13-17 erkannt werden, zur Identifizierung von Schizosaccharomyces.

7. Mittel zur Identifizierung von Schizosaccharomyces, welches Antikörper gegen Polypeptide oder immunogene Teile davon, welche von dem monoklonalen Antikörper JHF13-17 erkannt werden, enthält.

Im folgenden wird die Erfindung, insbesondere in ihren bevorzugten Ausführungsformen, detailliert beschrieben.

In einer immunoenzymatischen Methode, wie sie beispielsweise bei Burnette (Burnette W. N. (1981) Anal. Biochem. 112, 195-203) beschrieben ist, wurde unter Verwendung des murinen monoklonalen Antikörpers mAK JHF13-17 zwei Schizosaccharomyces-spezifische Polypeptide SSP-A und SSP-B aus S. pombe, S. malidevorans und S. japonicus gefunden. Mit demselben mAK wurden zwei weitere Schizosaccharomyces-spezifische Polypeptide SSP-B' und SSP-C aus S. octosporus gefunden. In folgenden Hefearten hingegen, die nicht zu der Gattung Schizosaccharomyces gehören, wurden die Polypeptide SSP-A, SSP-B, SSP-B' oder SSP-C nicht nachgewiesen: Bullera alba, Candida albicans, Candida krusei, Candida parapsilosis, Candida tropicalis, Cryptococcus neoformans, Cryptococcus terreus, Exobasidium japonicum, Filobasidium floriforme, Hansenula polymorpha, Kluyveromyces lactis, Leucosporidium nivale, Metschnikowia pulcherrima, Phodotorula graminis, Rhodotorula minuta, Rhodotorula rubra, Saccharomyces cerevisiae, Saccharomycodes ludwigii, Sporobolomyces holsaticus, Trichsporon cutaneum, Trigonopsis variabilis und Ustilago zeae.

Auch beispielsweise in Escherichia coli sowie in Ziegen- und Kaninchensera wurden die Schizosaccharomyces-spezifischen Polypeptide (SSP-A, SSP-B, SSP-B' und SSP-C) nicht nachgewiesen.

Die relative Molmasse der gefundenen Polypeptide wird im allgemeinen durch Vergleich mit Größenstandards bestimmt. Unter denaturierenden Bedingungen, beispielsweise in einem SDS-Polyacrylamidgel, wurden die Banden der Proteine SSP-A, SSP-B bzw. SSP-B' und SSP-C in einem Molmassenbereich von ca. 40.000 bis 60.000 nachgewiesen. Vorzugsweise wurden für die einzelnen Proteinbanden folgende relativen Molmassen bestimmt: SSP-A: 50.000 - 54.000, insbesondere ca. 52.000 SSP-B bzw.SSP-B': 48.000-52.000, insbesondere ca. 50.000 SSP-C: 46.000 - 50.000, insbesondere ca. 48.000

Die Erfindung betrifft ferner polyklonale Antikörper gegen SSP-A, SSP-B, SSP-B' und SSP-C, die nach bekannten Methoden, beispielsweise nach Coghlan L. G. & Hanausek M. (1990), J. Immunol. Meth. 129, 135-138, gewonnen werden können. Mit den gewonnenen monospezifischen polyklonalen Antikörpern kann gezeigt werden, daß nicht nur ein immunogener Teil bzw. Epitop des jeweiligen Proteins spezifisch für Schizosaccharomyces ist, sondern die Polypeptide SSP-A, SSP-B, SSP-B' und SSP-C als solche. Die Erfindung umfaßt schließlich auch mAK gegen die vorgenannten Polypeptide, die funktionell verschieden vom mAK JH13-17 sind und folglich andere Epitope der erfindungsgemäßen Polypeptide erkennen. Diese mAK werden nach allgemein bekannten Methoden, beispielsweise nach Ed Harlow and David Lane, eds. Antibodies. A Laboratory Manual Cold Spring Harbor Laboratory, Cold Spring Harbor, New York (1988), erzeugt. Im allgemeinen werden die monoklonalen oder polyklonalen Antikörper, insbesondere mAK JH13-17, zur Identifizierung der Hefegattung Schizosaccharomyces, beispielsweise in der Immunofluoreszenz, im Immunoblotting oder in ELISAs eingesetzt. Ferner können neben monoklonalen Antikörpern auch monospezifische polyklonale Antikörper zur Isolierung von SSP-A, SSP-B, SSP-B' und/oder SSP-C nach allgemein bekannten Methoden, beispielsweise Affinitätschromatographie, verwendet werden.

Mit Hilfe des mAK JHF13-17 können auch die Gene, die für SSP-A, SSP-B, SSP-B' oder SSP-C codieren, kloniert und die Identifizierung von Schizosaccharomyces über die spezifischen DNA oder RNA-Sequenzen vorgenommen werden. Im weiteren können die SSP-Gene oder die translatierenden Abschnitte in andere Hefen transformiert werden und hier als spezifische Marker dienen.

Der Nachweis von Schizosaccharomyces findet insbesondere in der Qualitätskontrolle, zum Beispiel bei der Alkoholproduktion aus Melasse, in der Weinherstellung oder bei der heterologen Produktion von Proteinen in Schizosaccharomyces Anwendung. Ein besonderer Vorteil ist der spezifische und sensitive Nachweis von Schizosaccharomyces. Daher können Hefehybriden, wie sie z. B. zwischen den Arten Saccharomyces cerevisiae und S. pombe hergestellt worden sind, eindeutig identifiziert werden.

Die Erfindung soll durch folgende Beispiele näher erläutert werden:

**Beispiel 1**

Verschiedene Mikroorganismen wurden 3 Tage in YPD-Medium (1 % Hefeextrakt, 2 % Pepton, 2 % Glucose) in 250 ml Erlenmeyerkolben auf einem Schüttelgerät bei 180 UpM und bei 15°C bzw. 30°C gezüchtet. Nach Zentrifugation von jeweils 10 ml Kulturbrühe wurden die Zellen geerntet, in 1 ml 10 mM Tris-Puffer (pH 7,5) aufgenommen, mit Glaskugeln versetzt und in einer Glaskugelmühle aufgeschlossen. Anschließend wurde das Homogenat nach Laemmli (U.K. Laemmli (1970) Nature 227, 680-685) erhitzt, in einem
10 %-igen SDS-Polyacrylamidgel aufgetrennt und auf Nitrozellulosemembranen transferiert (Burnette W. N. a.a.O.). Die Nitrozellulosestreifen wurden dann in Tris-Puffer (10 mM Tris-HCl, pH 7,5, 150 mM NaCl, 1 % Gelatine, 4 % Tween 29), die den mAK JHF13-17 (10 µg/ml) enthielt, inkubiert. Als Konjugat wurden anti-Maus Antikörper verwendet, die mit alkalischer Phosphatase gekoppelt waren. Als Substrate dienten Fast Blau B Salz (Fa. Serva, Heidelberg) und Naphtol AS-MX-Phosphat (Fa. Sigma Chemie, München).
Zur Bestimmung der relativen Molmasse wurden auf dem SDS-Polyacrylamidgel zusätzlich Rainbow™ Protein Molekulargewichtsmarker (Amersham Buchler GmbH, Braunschweig) aufgetragen.

In den folgenden Schizosaccharomyces-Stämmen wurden mit mAK JHF13-17 schizosaccharomyces-spezifische Proteine nachgewiesen. (Liste 1)

| Organismus | Stammsammlung |
|---|---|
| S. pombe | CBS 5680 |
| | CBS 1057 |
| | DSM 3796 (leu, his)[a] |
| | CSIR Y-467 |
| | CBS 1043 |
| | CBS 1043 |
| S. malidevorans | NCYC 683 |
| | VLSF 0401 |
| | CBS 3557 |
| | BLWG 442 |
| S. japonicus var. japonicus | VLSF 03/101 |
| | VLSF 03/201 |
| | IFO 1609 |
| | IFO 1646 |
| | IFO 1712 |
| S. japonicus var. versatilis | NCYC 419 |
| | IFO 1607 |
| S. octosporus | NCYC 131 |
| | VLSF 0101 |
| | CBS 2631 |
| | CBS 1804 |
| | CBS 2632 |
| | CBS 371 |
| | CBS 6209 |
| | BLWG H80 |
| | ATCC 2479 |

[a] = genetischer Marker

Abkürzungsverzeichnis der Stammsammlungen:

The Centraalbureau voor Schimmelcultures (CBS), Baarn, The Netherlands; Deutsche Stammsammlung für Mikroorganismen und Zellkulturen GmbH (DSM), Braunschweig, Bundesrepublik Deutschland; Versuchs- und Lehranstalt für Spiritusfabrikation und Fermentationstechnologie in Berlin (VLSF), Berlin, Bundesrepublik Deutschland; Institute for Fermentation (IFO); Osaka, Japan; Council of Science of Industrial Research (CSIR), Pretoria, South Africa; National Collection of Yeast Cultures (NCYC), Norwich, England; Bayerische Landesanstalt für Weinbau und Gartenbau (BLWG), Würzburg, Bundesrepublik Deutschland; American Type Culture Collection (ATCC), Rockville, Maryland, USA.

In den Zellextrakten von S. pombe, S. malidevorans und S. japonicus war jeweils eine Doppelbande sichtbar, die eine relative Molmasse von ca. 52.000 (SSP-A) und ca. 50.000 (SSP-B) aufwiesen. Die elektrophoretische Mobilität der Doppelbande war in allen untersuchten Stämmen identisch. Auf Grund der Kreuz-Reaktivität mit dem mAK JHF13-17 sind die beiden Proteine wahrscheinlich miteinander verwandt.

In Zellextrakten von S. octosporus wurde ebenfalls eine Doppelbande nach der oben beschriebenen Methode sichtbar. Allerdings unterscheidet sich die elektrophoretische Mobilität dadurch, daß die obere Proteinbande (SSP-B') in Höhe von SSP-B aus S. pombe, S. malidevorans und S. japonicus wandert. Das zweite Protein von S. octosporus (SSP-C) besitzt eine relative Molmasse von ca. 48.000. In den untersuchten Stämmen von S. octosporus war die elektrophoretische Mobilität der Doppelbande identisch.

**Beispiel 2**

Um die Frage zu klären, ob die mit mAK JHF13-17 reagierenden Proteine SSP-A, SSP-B und SSP-C spezifisch für Spalthefen sind, wurden Zellextrakte von 23 anderen Hefearten nach der in Beispiel 1 beschriebenen Methode untersucht (Liste 2). Die getesteten Arten waren:

Bullera alba DSM 70002

Candida albicans

Candida krusei

Candida parapsilosis

Candida tropicalis

Cryptococcus neoformans

Cryptococcus terreus DSM 70222

Exobasidium japonicum DSM 4461

Filobasidium floriforme DSM 4655

Hansenula polymorpha (leu1)[a]

Kluyveromyces lactis SD11 (lac4, trp1)[a]

Leucosporidium nivale DSM 4635

Metschnikowia pulcherrima DSM 70879

Rhodotorula graminis

Rhodotorula minuta

Rhodotorula rubra

Saccharomyces cerevisiae VLSF 104

Saccharomyces cerevisiae (ura3-2, leu2, his, pra1, prb1, prc1, cps1)[a]

Saccharomycodes ludwigii DSM 3447

Sporobolomyces holsaticus DSM 70580

Sterigmatomyces halophilus DSM 4668

Trichosporon cutaneum DSM 70698

Trichosporon cutaneum

Trigonopsis variabilis DSM 70714

Ustilago zeae DSM 4500

Das Ergebnis war, daß in keiner dieser Hefearten ein Protein mit dem mAK JHF13-17 spezifisch nachgewiesen wurde. Auch Zellextrakte von Escherichia coli sowie Ziegen- und Kaninchenserum enthielten kein Protein, das von dem mAK JHF13-17 spezifisch erkannt wurde. Das von mAK JHF13-17 auf den Proteinen SSP-A, SSP-B, SSP-B' und SSP-C erkannte Epitop ist somit spezifisch für Spalthefen.

**Beispiel 3**

Um zu zeigen, daß nicht nur ein Epitop der Schizosaccharomyces-spezifischen Proteine (SSPs), die der mAK JHF13-17 erkennt, spezifisch ist, sondern das Protein als solches spezifisch für Schizosaccharomyces ist, wurde ein polyklonales Serum gegen SSP-A und SSP-B von S. pombe hergestellt. Dazu wurden nach der in Beispiel 1 beschriebenen Methode die Proteine eines S. pombe-Zellextraktes auf Nitrozellulose übertragen und der Bereich ausgeschnitten, der der Lage des SSP-A und SSP-B entspricht. Anschließend wurden Kaninchen mit diesen Nitrozellulose-gebundenen SSP-A und SSP-B nach der Methode von Coglan und Hanausek (J. Immunol. Meth. 129, 135-138 [1990]) immunisiert. Die gewonnenen monospezifischen, polyklonalen Antisera reagierten in Western blot-Analysen nur mit den SSPs der vier Arten von Schizosaccharomyces (SSP-A, SSP-B, SSP-B' und SSP-C), nicht aber mit anderen Zellextrakt-Proteinen oder mit Proteinen von anderen Hefearten. Folglich sind vorgenannte SSPs spezifisch für Spalthefen.

**Patentansprüche**

**Patentansprüche für folgende Vertagsstaaten : AT, BE, CH, DE, DK, FR, GB, IT, LI, LU, NL, PT, SE**

1. Polypeptide oder immunogene Teile davon aus Schizosaccharomyces, dadurch gekennzeichnet, daß sie von dem monoklonalen Antikörper JHF13-17 (DSM ACC 2005) erkannt werden.

2. Polypeptide nach Anspruch 1, dadurch gekennzeichnet, daß sie unter denaturierenden Bedingungen eine relative Molmasse von ca. 40.000 - 60.000 haben.

3. Polypeptide nach einem der Ansprüche 1 oder 2, dadurch gekennzeichnet, daß sie aus S. pombe, S. malidevorans, S. japonicus oder S. octosporus stammen.

4. Monoklonale Antikörper gegen Polypeptide oder immunogene Teile davon nach Anspruch 1.

[a] = genetischer Marker

**5.** Monoklonaler Antikörper nach Anspruch 4, dadurch gekennzeichnet, daß es der monoklonale Antikörper JHF13-17 (DSM ACC 2005) ist.

**6.** Polyklonale Antikörper gegen Polypeptide nach Anspruch 1.

**7.** Verfahren zur Herstellung von polyklonalen Antikörpern, dadurch gekennzeichnet, daß
a) Polypeptide oder immunogene Teile davon aus Schizosaccharomyces, welche von dem monoklonalen Antikörper JHF13-17 (DSM ACC 2005) erkannt werden, isoliert werden und
b) ein geeigneter Organismus mit Polypeptiden oder immunogenen Teilen davon aus Schritt a) immunisiert wird.

**8.** Verfahren zur Identifizierung von Schizosaccharomyces, dadurch gekennzeichnet, daß Schizosaccharomyces zusammen mit Antikörper gegen Polypeptide oder immunogene Teile davon nach Anspruch 1 inkubiert wird.

**9.** Verfahren nach Anspruch 8, dadurch gekennzeichnet, daß die Antikörper polyklonal oder monoklonal sind.

**10.** Verfahren nach einem der Ansprüche 8 oder 9, dadurch gekennzeichnet, daß der monoklonale Antikörper JHF13-17 (DSM ACC 2005) verwendet wird.

**11.** Verwendung von Antikörper gegen Polypeptide oder immunogene Teile davon nach Anspruch 1 zur Identifizierung von Schizosaccharomyces.

**12.** Mittel zur Identifizierung von Schizosaccharomyces, welches Antikörper gegen Polypeptide oder immunogene Teile davon nach Anspruch 1 enthält.

**Patentansprüche für folgende Vertagsstaaten : ES, GR**

**1.** Verfahren zur Identifizierung von Schizosaccharomyces, dadurch gekennzeichnet, daß Schizosaccharomyces mit Antikörper gegen Polypeptide oder immunogene Teile davon, die von dem monoklonalen Antikörper JHF13-17 (DSM ACC 2005) erkannt werden, in Kontakt gebracht wird.

**2.** Verfahren nach Anspruch 1, dadurch gekennzeichnet, daß die Polypeptide unter denaturierenden Bedingungen eine relative Molmasse von ca. 40.000 - 60.000 haben.

**3.** Verfahren zur Identifizierung von Schizosaccharomyces, dadurch gekennzeichnet, daß Schizosaccharomyces zusammen mit dem Antikörper JHF13-17 (DSM ACC 2005) in Kontakt gebracht wird.

**4.** Verfahren nach einem der Ansprüche 1 bis 3, dadurch gekennzeichnet, daß Schizosaccharomyces S. pombe, S. malidevorans, S. japonicus oder S. octosporus ist.

**5.** Verfahren zur Herstellung von polyklonalen Antikörpern, dadurch gekennzeichnet, daß
a) Polypeptide oder immunogene Teile davon aus Schizosaccharomyces, welche von dem monoklonalen Antikörper JHF13-17 (DSM ACC 2005) erkannt werden, isoliert werden und
b) ein geeigneter Organismus mit Polypeptiden oder immunogenen Teilen davon aus Schritt a) immunisiert wird.

**6.** Verwendung von Antikörper gegen Polypeptide oder immunogene Teile, die von dem monoklonalen Antikörper JHF 13-17 (DSM ACC 2005) erkannt werden, zur Identifizierung von Schizosaccharomyces.

**Claims**
**Claims for the following Contracting States : AT, BE, CH, DE, DK, FR, GB, IT, LI, LU, NL, PT, SE**

**1.** A polypeptide, or an immunogenic portion thereof, from Schizosaccharomyces, which is recognized by the monoclonal antibody JHF13-17 (DSM ACC 2005).

2. The polypeptide as claimed in claim 1, which has a relative molecular weight of approx. 40,000 - 60,000 under denaturating conditions.

3. The polypeptide as claimed in one of claims 1 or 2, which is derived from S. pombe, S. malidevorans, S. japonicus or S. octosporus.

4. A monoclonal antibody against a polypeptide, or an immunogenic portion thereof, as claimed in claim 1.

5. The monoclonal antibody as claimed in claim 4, it being the monoclonal antibody JHF13-17 (DSM ACC 2005).

6. A polyclonal antibody against a polypeptide as claimed in claim 1.

7. A process for the preparation of a polyclonal antibody, which comprises
   a) isolating a polypeptide, or an immunogenic portion thereof, from Schizosaccharomyces, which is recognized by the monoclonal antibody JHF13-17 (DSM ACC 2005), and
   b) immunizing a suitable organism with a polypeptide, or an immunogenic portion thereof, from step a).

8. A process for identifying Schizosaccharomyces which comprises incubating Schizosaccharomyces together with an antibody against a polypeptide, or an immunogenic portion thereof, as claimed in claim 1.

9. The process as claimed in claim 8, wherein the antibody is polyclonal or monoclonal.

10. The process as claimed in one of claims 8 or 9, wherein the monoclonal antibody JHF13-17 (DSM ACC 2005) is used.

11. The use of an antibody against a polypeptide, or an immunogenic portion thereof, as claimed in claim 1 for identifying Schizosaccharomyces.

12. A composition for identifying Schizosaccharomyces, comprising an antibody against a polypeptide, or an immunogenic portion thereof, as claimed in claim 1.

**Claims for the following Contracting States : ES, GR**

1. A process for identifying Schizosaccharomyces, which comprises bringing Schizosaccharomyces into contact with an antibody against a polypeptide, or an immunogenic portion thereof, which is recognized by the monoclonal antibody JHF13-17 (DSM ACC 2005).

2. The process as claimed in claim 1, wherein the polypeptide has a relative molecular weight of approx. 40,000 - 60,000 under denaturating conditions.

3. A process for identifying Schizosaccharomyces, which comprises bringing Schizosaccharomyces into contact with the antibody JHF13-17 (DSM ACC 2005).

4. The process as claimed in claim 1 to 3, wherein Schizosaccharomyces is S. pombe, S. malidevorans, S. japonicus or S. octosporus.

5. A process for the preparation of a polyclonal antibody, which comprises
   a) isolating a polypeptide, or an immunogenic portion thereof, from Schizosaccharomyces, which is recognized by the monoclonal antibody JHF13-17 (DSM ACC 2005), and
   b) immunizing a suitable organism with a polypeptide, or an immunogenic portion thereof, from step a).

6. The use of antibody against a polypeptide, or an immunogenic portion thereof, which is recognized by the monoclonal antibody JHF13-17 (DSM ACC 2005), for identifying Schizosaccharomyces.

**Revendications**
**Revendications pour les Etats contractants suivants : AT, BE, CH, DE, DK, FR, GB, IT, LI, LU, NL, PT, SE**

1. Polypeptides ou fragments immunogènes de ceux-ci, provenant de *Schizosaccharomyces,* caractérisés en ce qu'ils sont reconnus par l'anticorps monoclonal JHF13-17 (DSM ACC 2005).

2. Polypeptides selon la revendication 1, caractérisés en ce que, dans des conditions dénaturantes, ils ont une masse moléculaire relative de 40 000 - 60 000.

3. Polypeptides selon la revendication 1 ou 2, caractérisés en ce qu'ils proviennent de *S. pombe, S. malidevorans, S. japohicus* ou *S. octosporus.*

4. Anticorps monoclonal dirigé contre des polypeptides, ou des fragments immunogènes de ceux-ci, selon la revendication 1.

5. Anticorps monoclonal selon la revendication 4, caractérisé en ce qu'il est l'anticorps monoclonal JHF13-17 (DSM ACC 2005).

6. Anticorps polyclonal dirigé contre des polypeptides selon la revendication 1.

7. Procédé pour la production d'anticorps polyclonaux, caractérisé en ce que
   (a) on isole des polypeptides ou des fragments immunogènes de ceux-ci, provenant de *Schizosaccharomyces*, qui sont reconnus par l'anticorps monoclonal JHF13-17 (DSM ACC 2005) et
   (b) on immunise avec des polypeptides ou des fragments immunogènes de ceux-ci, provenant de l'étape a), un organisme approprié.

8. Procédé pour l'identification de *Schizosaccharomyces,* caractérisé en ce que l'on met *Schizcsac−charomyces* à incuber avec des anticorps dirigés contre des polypeptides ou des fragments immunogènes de ceux-ci, selon la revendication 1.

9. Procédé selon la revendication 8, caractérisé en ce que les anticorps sont polyclonaux ou monoclonaux.

10. Procédé selon la revendication 8 ou 9, caractérisé en ce qu'on utilise l'anticorps monoclonal JHF13-17 (DSM ACC 2005).

11. Utilisation d'anticorps dirigés contre des polypeptides ou des fragments de ceux-ci, selon la revendication 1, pour l'identification de *Schizosaccharomyces.*

12. Agent pour l'identification de *Schizosaccharomyces,* qui contient des anticorps dirigés contre des polypeptides ou des fragments immunogènes de ceux-ci, selon la revendication 1.

**Revendications pour les Etats contractants suivants : ES, GR**

1. Procédé pour l'identification de *Schizosaccharomyces,* caractérisé en ce que l'on met *Schizosaccharomyces* en contact avec des anticorps dirigés contre des polypeptides ou des fragments immunogènes de ceux-ci, qui sont reconnus par l'anticorps monoclonal JHF13-17 (DSM ACC 2005).

2. Procédé selon la revendication 1, caractérisé en ce que, dans des conditions dénaturantes, les polypeptides ont une masse moléculaire relative de 40 000 - 60 000.

3. Procédé pour l'identification de *Schizosaccharomyces,* caractérisé en ce que l'on met *Schizosaccharomyces* en contact avec l'anticorps JHF13-17 (DSM ACC 2005).

4. Procédé selon l'une des revendications 1 à 3, caractérisé en ce que *Schizosaccharomyces* est *S. pombe, S. malidevorans, S. japonicus* ou *S. octosporus.*

5. Procédé pour la production d'anticorps polyclonaux, caractérisé en ce que
   (a) on isole des polypeptides ou des fragments immunogènes de ceux-ci, provenant de *Schizosaccharomyces,* qui sont reconnus par l'anticorps monoclonal JHF13-17 (DSM ACC 2005) et
   (b) on immunise avec des polypeptides ou des fragments immunogènes de ceux-ci, provenant de l'étape a), un organisme approprié.

6. Utilisation d'anticorps dirigés contre des polypeptides ou des fragments de ceux-ci, qui sont reconnus par l'anticorps monoclonal JHF 13-17 (DSM ACC 2005), pour l'identification de *Schizosaccharomyces.*